# EUROPEAN PATENT SPECIFICATION

(11) **EP 0 896 509 B1**
(45) Date of publication and mention of the grant of the patent: **27.06.2007**
(21) Application number: 97915762.5
(22) Date of filing: 15.04.1997
(51) Int. Cl.: A23C 19/032, C12N 15/57, C12N 15/75

(54) **METHODS FOR PRODUCING DAIRY PRODUCTS, IN PARTICULAR CHEESE USING LACTIC ACID BACTERIA PROVIDED WITH ADDITIONAL NEUTRAL PROTEASE ACTIVITY**
VERFAHREN ZUR HERSTELLUNG VON MILCHPRODUKTEN, INSBESONDERE KÄSE, UNTER VERWENDUNG VON MILCHSÄUREBAKTERIEN DIE MIT ZUSÄTZLICHER NEUTRALER PROTEASEAKTIVITÄT AUSGESTATTET SIND
PROCEDE DE PRODUCTION DE PRODUITS LAITIERS, NOTAMMENT DE FROMAGE, A PARTIR DE BACTERIES D'ACIDE LACTIQUE POSSEDANT UNE ACTIVITE COMPLEMENTAIRE DE PROTEASE NEUTRE

(30) Priority: 15.04.1996 EP 96200948
(43) Date of publication of application: 17.02.1999
(73) Proprietor: Friesland Brands B.V., 7943 PE Meppel (NL)
(72) Inventor: MIERAU, Igor, NL-9728 AL Groningen (NL); VAN DEN BURG, Lambertus, NL-8161 HD Epe (NL); KOK, Jan, NL-9714 HL Groningen (NL); VENEMA, Gerhardus, NL-9751 NN Haren (NL)
(74) Representative: Smulders, Theodorus A.H.J.
(86) International application number: PCT/NL1997/000192
(87) International publication number: WO 1997/038587

(56) References cited:
- JOURNAL OF DAIRY SCIENCE, vol. 76, 1993, CHAPAIGN, ILLINOIS US, pages 2133-2144, XP000606357 G. VENEMA: "Molecular biology and genetic modification of lactococci"
- APPLIED AND ENVIRONMENTAL MICROBIOLOGY, vol. 56, no. 9, 1990, pages 2606-2611, XP000603149 M. VAN DE GUCHTE: "Heterologous gene expression in Lactococcus lactis subsp. lactis" cited in the application
- APPLIED AND ENVIRONMENTAL MICROBIOLOGY, vol. 60, no. 12, 1994, pages 4226-4233, XP000603247 A. MCGARRY: "Effect of genetically modyfying the lactococcal proteolytic system on ripening and flavor development in cheddar cheese" cited in the application
- JOURNAL OF DAIRY SCIENCE, vol. 77, no. 8, 1994, CHAPAIGN, ILLINOIS US, pages 2150-2159, XP000468028 H. RIEPE: "Oversecration of the neutral protease from Bacillus subtilis in Lactococcus lactis spp. lactis JF254"
- JOURNAL OF DAIRY SCIENCE, vol. 76, no. 7, 1993, CHAPAIGN, ILLINOIS US, pages 2056-2064, XP002034378 J. KOK: "Genetics of proteolytic enzymes of lactococci and their role in cheese flavour development"
- JOURNAL OF CHEMICAL TECHNOLOGY AND BIOTECHNOLOGY, vol. 58, no. 2, 1993, OXFORD GB, pages 195-199, XP000388270 G. FITZGERALD: "Molecular manipulations of Lactococcus starter cultures for food fermentations"
- JOURNAL OF DAIRY SCIENCE, vol. 76, no. 9, 1993, CHAPAIGN, ILLINOIS US, pages 2455-2467, XP000396656 J. LAW: "The contribution of Lactococcal starter proteinases to proteolysis in cheddar cheese"
- VOEDINGSMIDDELEN TECHNOLOGIE, vol. 20, no. 1, 1987, ZEIST NL, pages 24-27, XP002015038 A. SIMONS: "Genetisch onderzoek van melkzuurbacterien"

## Description

The invention relates to processes for making fermented food products from raw starting materials such as milk, meat, cereals and vegetables.

Fermenting these materials is often carried out using so called lactic acid bacteria. Lacic acid bacteria include members of the genera *Lactobacillus, Lactococcus, Leuconostoc, Pediococcus* and *Streptococcus.* The fermenting step is carried out for a number of reasons. It helps to enhance the time over which the food product can be preserved and it can add to the development of flavour and texture of the product. In addition, some fermented milk products are stated to have health and nutritional benefits.

The invention especially relates to processes for making cheese.

Processes for making cheese are of course different for each different kind of cheese to be made. In principle however there are a number of main steps in the process which are essentially the same.

First of all to the milk (be it full milk, or skimmed milk, pasteurized ot not) a starter culture, rennet and usually calcium are added to start coagulation. After an appropriate time interval, during which the coagulum is cut and stirred, allowing for sufficient syneresis the whey is drained off.

The remaining material is called the curd. The curd has to ripen into cheese, but this is where the processes start to vary, depending on the kind of cheese to be made. In the case of gouda type cheese, the curd is pressed in a cheese vat (or mould) and then transferred to the brine. The cheeses are left to soak in the brine for a time interval in the order of days and are then transferred to the warehouse for further ripening.

Thus from the moment that the cheese is pressed there is hardly any possibility to add anything to the cheese. Cheddar type cheeses are brought into contact with the salt before pressing, during milling of the curd. Thus for cheddar cheese it is possible to add substances until a later moment in the cheese making process. The lactic acid bacteria are usually present in the starting culture. Lactic acid bacteria convert lactose into lactic acid, but they also produce proteolytic enzymes that degrade proteins present in the milk. The activity of the lactic acid bacteria is present throughout the whole process. The proteolytic activity especially, is very important for the ripening of the cheese. It determines for a large part the taste and texture characteristics of the resulting cheese. Therefore it is desirable to be able to design the rate of ripening and the extent of ripening that a cheese will be subjected to.

It has been suggested to enhance the rate of ripening by adding proteolytic enzymes during various stages of the process. When enzymes are added to the milk, only a small fraction thereof is retained in the curd (El Soda, 1990). This means that a relatively high amount of expensive enzyme preparations have to be added. Moreover, even in processes where the enzymes may be added later on, for instance to the curd, it will be hard, if not impossible to ensure an even distribution of the enzyme through the curd.

A way to provide for a more even distribution of materials (especially brine) through the curd has been suggested in EP-A-0 492 716. Herein injection of liquids under high pressure into the curd or cheese are disclosed. This process seems rather complicated and expensive and rather unsuitable for enzymes or microorganisms, which are likely to degrade under high pressure, because of for instance shear forces.

It remains a desire, for many reasons disclosed herein, to be able to provide the curd with additonal enzyme activity which is evenly distributed through the curd. Additions of *B.subtilis* neutral protease preparations were shown to contribute in a significant amount to the degradation of proteins present in cows or ewe's milk during ripening of cheddar cheese (Fernandez-Garcia et al.,1990). However, as stated before, an equal distribution of enzyme through the curd, and thus an equal rate of ripening is very hard to obtain in that manner.

A possible way to ensure equal distribution of the ripening proteolytic activity through the curd, is by providing the lactic acid bacteria of the starter culture with the wanted proteolytic activity. This can be accomplished by introducing the gene encoding the enzyme of interest in said lactic acid bacteria. Using a heterologous gene expression vector van der Guchte et *al.* (1989, 1990, 1991, 1992a, 1992b) obtained expression of several heterologous genes in *L. lactis,* including the *Bacillus subtilis* neutral protease gene and the gene of hen egg white lysozyme. The contribution of the *B. subtilis npr* gene product, as expressed by *L. lactis,* to the ripening and flavor development of Cheddar cheese was tested by McCarry *et al*. (1994). Their results showed that cheeses produced with *L. lactis* harboring the *B. subtilis npr* gene, on the expression vector constructed by van der Guchte *et al.* (1990) were subject to greatly accelerated proteolysis, resulting in an overripening of the cheeses within one month.

Riepe et al (Journal of dairy Science, Vol 77, 1994, p 2150-2159) constructed a lactococcal strain that oversecretes B. subtilis neutral protease. The oversecreting neutral protease transformant exhibited a halo of white precipitate when it was grown on transparent citrate milk agar and completely solubilized 11% Nom at 21°C after 5 days.

The invention provides a method for carrying out a process of biotransformation of a substrate whereby at least one lactic acid bacterium is used, said bacterium comprising a gene encoding a bacillus subtilis neutral protease having an altered resulting activity. For instance by using a lactic acid bacterium which has a neutral protease gene of more limited stability, especially in processes of making cheese, it is possible to get enhanced ripening of the cheese, but to limit said enhancement in time and rate. Instead of altering the duration of the activity of the neutral protease, it is of course also very suitable to provide a neutral protease which has a lower activity for a longer period of time, or to regulate the expression of the neutral protease so that the activity can be switched on or off more or less at will.

It is also very useful to provide Bacillus subtilis neutral proteases with altered specificity, as also disclosed in the present invention. The important aspect is that bacteria are provided which have a Bacillus subtilis neutral protease activity which is especially designed for their application. Therefore combinations of mutations in Bacillus subtilis neutral protease genes are preferred embodiments of the present invention. For instance, it may be very useful to have a highly active Bacillus subtilis neutral protease with a very limited stability, or a mildly active Bacillus subtilis neutral protease with prolonged stability, or vice versa depending on the specific application. All altered Bacillus subtilis neutral protease activity and/or specificity and/or stability are preferably under control of an inducible mechanism for expression, preferably an inducible promoter. Especially preferred is a system whereby the Bacillus subtilis neutral protease has different activities/stabilities, preferably under control of a regulatable mechanism, are used or are available for use when necessary. By having an array of different stabilities of Bacillus subtilis neutral protease activity available, it is possible to regulate and monitor the cheese ripening process, or other proteolytic biotransformation processes for that matter, in detail. Therewith the taste characteristics of the cheese can also be influenced with the required subtileness. It will be clear that for many applications according to the invention it may not be necessary to provide the lactic acid bacterium with a complete gene encoding the relevant protease. Parts thereof resulting in a polypeptide having the same kind of activity may of course be used. These parts may even be specifically chosen for reasons of a different stability or half-life. Variants of the exemplified proteases are also part of the invention. They may be also specifically chosen for their different properties in terms of stability, activity and half-life. Some preferred mutations of Bacillus subtilis proteases include at least one of the following
: mutation of the codon encoding leucine at position 300;
: deletion of the codon encoding leucine at position 300;
:mutation of the codons for residues 120, 134 and/or 136;
: mutation of the codons 186 and 194 to cysteine residues; or
: mutation of the codons 102 and 120 to cysteine residues.

Suitable expression and/or other regulatory elements should be provided with the gene to be expressed by the lactic acid bacteria. Suitable,elements for regulated expression of the Bacillus subtilis neutral proteases according to the invention include, but are not limited to the prtP promoter, which can be induced by specific dipeptides; the nisin promoter, which can be induced by nisin; the superoxide dismutase promoter, which can be induced by oxygen; the LacZ promoter, inducible by IPTG; and T7 polymerase, inducible by lactose. These elements are well-known in the art. Preferred are regulatory elements which are derived from lactic acid bacteria themselves, because they are usually best suited for expression in those bacteria. Most preferred are autologous regulatory elements, such as the prtP, the nisin and the SOD promoter. For transferring the gene and the regulatory elements into the host cell many vehicles are now known. These vehicles may all be used according to the present invention. Preferred are those transfer vehicles which are designed to integrate the relevant gene into the host genome, without integration of any material from the transferring vehicle.

Recently developed procedures have made it possible to insert genes of interest into the chromosome of *L. lactis* at desired positions, and in such a way that, apart from the heterologous gene, no additional DNA sequences are incorporated. As a result, very well defined genetically modified strains can be obtained, that are equipped with additional genetic information, resulting in a microorganism with improved enzymatic properties. Although these procedures were initially developed for use in *L. lactis,* they are applicable to other lactic acid bacteria as well. Examples thereof are known and given herein below. The choice of the host cell to be provided with the additional protease activity depends on the kind of process of biotransformation to be carried out. For making cheese it should be a lactic acid bacterium which can be added to or is part of the starter culture. A very suitable genus of lactic acid bacteria is the *Lactococcus* genus.

It is possible according to the invention to produce an array of different lactic acid bacteria having additional protease activity, all of which may have different activities, stabilities and/or half-lifes. It is of course also possible to provide a single lactic acid bacterium with different protease activities. By providing such a set of different protease activities, it is possible to fine tune the ripening process of cheese or to fine tune other biotransformation processes. Therefor it is possible to obtain for instance cheeses within a shortened ripening time interval, but it is also possible to obtain cheeses with different taste characteristics. These products which were not obtainable until the present invention are also part thereof.

The invention will now be explained in more detail in the following detailed description.

### Detailed description.

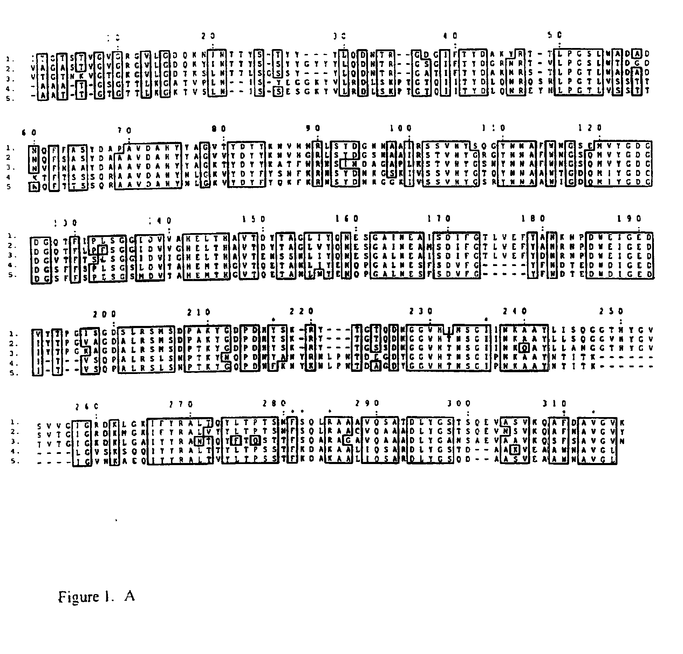

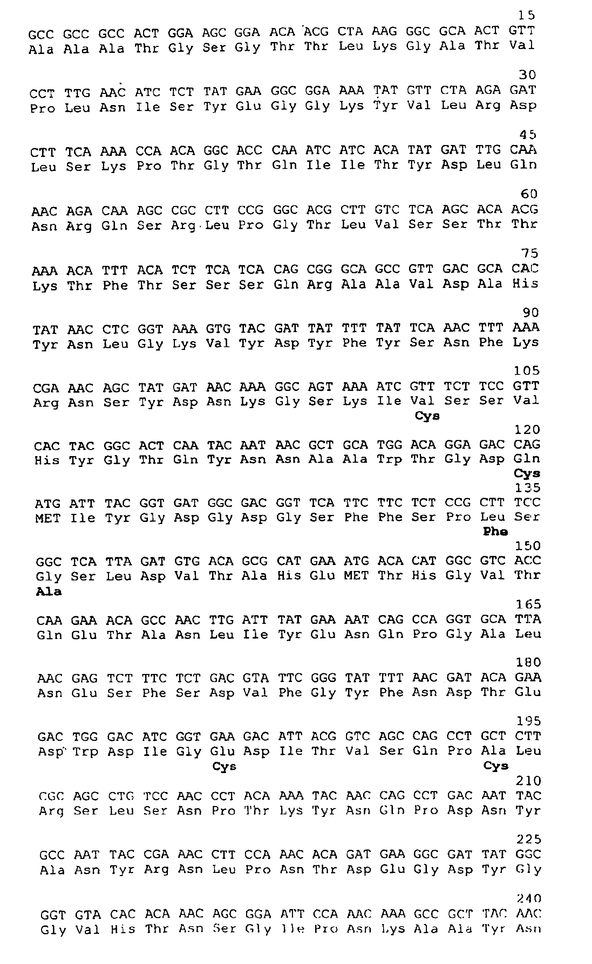

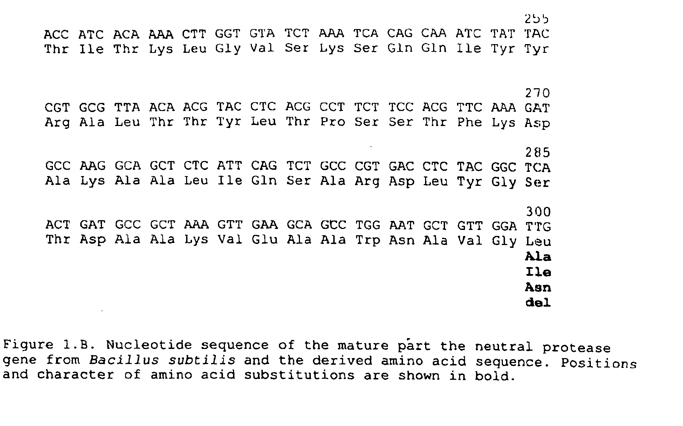

The neutral proteases (NP's) constitute a group of extracellular metallo-endopeptidases that are produced by several members of the genus Bacillus. Representatives of this class are used in several industrial processes, the most important of which is the preparation of the artificial sweetener aspartame (Gerhartz, 1990; Isowa *et al.,* 1979). NP's are also employed in the leather and baking industry, in breweries, and in the production of protein hydrolysates (Gerhartz, 1990). At present, thermolysin (e.g. in the preparation of aspartame) and the neutral protease of *Bacillus subtilis* (e.g. for beer-brewing applications) are the most frequently used NP's in industrial processes. Several Bacilli are known to produce NP's. These enzymes contain 300-319 amino acid residues and are active in the neutral pH range. The best known is thermolysin, the highly thermostable 316-residue NP from *B. thermoproteolyticus. Bacilli* exhibit considerable differences in optimal growth temperatures and the thermostabilities of their neutral proteases differ accordingly. Several *Bacillus* neutral proteases have been characterized and genes coding for these enzymes have been cloned and sequenced from e.g. *B. subtilis* (Yang *et al.,* 1984), *B. stearothermophilus* CU-21 (Fujii *et al.,* 1983; Takagi *et al.,* 1985), *B. stearothermophilus* MK-232 (Kubo & Imanaka, 1988), *B. thermoproteolyticus rokko* (Marquardt *et al*., 1990), and *B. caldolyticus* (Van den Burg *et al.,* 1991). Alignment of the amino acid sequences of the NP's shows a high level of sequence similarity between the different representatives (Figure 1.a). Using genetic techniques (such as 'site directed mutagenesis', e.g. Stanssens *et al.,* 1989), genes encoding NP's have been mutated to change properties of the enzymes, such as thermal stability, activity and specificity (Imanaka *et al.,* 1986; Toma *et al*., 1991; van den Burg *et al,* 1991; Eijsink *et al*., 1991; Kubo *et al.,* 1992). These experiments have led to a better understanding of the mechanisms that influence thermostability of NP's. Application of this knowledge has resulted in the construction of mutant NP's, characterized by thermostabilities exceeding that of any of the known NP's (Eijsink *et al.,* 1995). Additionally mutant NP's have been constructed that are characterized by a decreased thermostability. Availability of thermostable variants is desirable, since it expands the temperature range at which NP-catalysed processes can be conducted and enzyme activity lasts longer, reducing the amount of enzyme required. Variants with reduced thermostabilities can be useful in applications where enzymatic activity is only required during the initial stages of the process, or where it can be stopped by a simple heat treatment. Thermostability of NP's is, at least under experimental conditions, determined by the rate at which local unfolding processes occur, rendering the molecules susceptible to autolysis (Eijsink *et al*., 1991c, 1992c; Vriend and Eijsink, 1993; Dahlquist *et al.,* 1976; Braxton and Wells, 1992). As a consequence, thermostable variants are more resistant towards autoproteolysis, whereas thermolabile NP's are more vulnerable towards autoproteolytic breakdown.

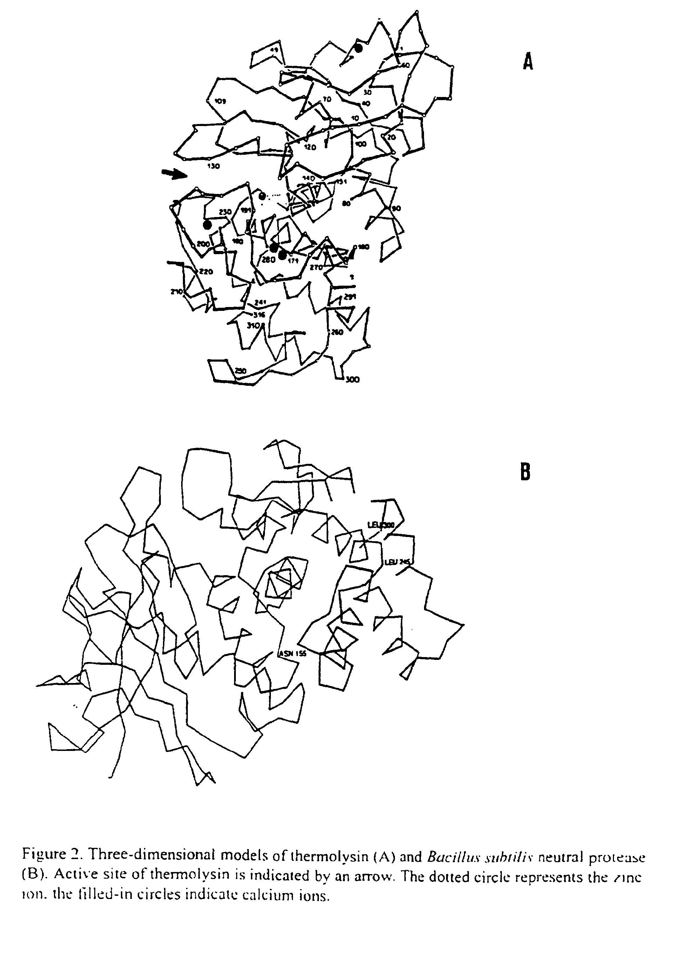

The three dimensional structure of thermolysin has been solved at 2.3 Å (Figure 2.a; Matthews *et al.,* 1972a, b; Colman *et al.,* 1972). The enzyme contains one zinc ion and four calcium ions, which are needed for activity and stability, respectively. The molecule is generally considered to consist of two domains, interconnected by a central α-helix (residues 137-150). The C-terminal domain may be divided into two subdomains connected by the 235-247 α-helix (Fontana, 1988; Eijsink *et al.,* 1992). In the active site cleft (indicated by an arrow in Figure 2.a) the zinc ion is bound. It is coordinated by the side chains of Glu166, His142, His146, and a water molecule, and these residues are conserved in all *Bacillus* NP's. The zinc ion and the fully conserved Glu143, His231 and, to a lesser extent, Tyrl57 play an important role in the catalytic mechanism (Kester and Matthews, 1977; Hangauer et *al* , 1984; Tronrud *et al* ., 1986). The catalytic mechanism of thermolysin is well established owing to extensive crystallographic studies of complexes between this enzyme and specific inhibitors (Hangauer *et al*., 1984; Tronrud *et al*., 1986; Toma *et al* ., 1989; Dunn, 1989) . The conservation among the NP' s of those residues that are involved in catalysis in thermolysin suggests that all neutral proteases have the same catalytic mechanism. This supposition is strongly supported by crystallographic analysis of the *B. cereus* NP (Pauptit *et al*., 1988) which showed an arrangement of presumed active site residues similar to that in thermolysin. Site-directed mutagenesis experiments have confirmed the importance of the residues Glu143 and His231 for catalysis in the *B. subtilis* NP. During catalysis the zinc coordinated water molecule is forced in the direction of the Glu143 side chain by the incoming substrate. As a result, the nucleophility of the water molecule is enhanced resulting in a nucleophilic attack on the carbonyl carbon of the scisile bond, resulting in cleavage. Residue His231 is important in stabilizing the transition state of the proteolytic reaction. Specificity and activity of the enzyme is additionally determined by amino acid residues in the substrate binding sites S₁, S₂, S₁' and S₂' (Hangauer *et al.,* 1984; Matthews *et al.,* 1988). Substrate specificities of NP's are, furthermore, determined by residues constituting the entrance of the active site cleft. The presence of specific large and/or charged residues at particular positions can restrict the sizes of the substrates still able to enter. Thus, substitution of specific residues constituting the active site entrance (e.g. residue 120 in *B. subtilis* NP) can increase the number of substrates that can reach the active site.

The present invention includes the use of variants of the *B*. *subtilis* neutral protease displaying altered activities and specificities, as obtained by site-directed mutagenesis of specific residues involved in the interaction between the enzyme and substrates (Gln120 and Phe134). Mutations affecting the so-called hinge bending of the NP's are also included. Hinge bending, the motion of the N-terminal (residues 1-150) and C-terminal domains of NP relative to each other, can be manipulated by mutations in the interconnecting α-helix (137-150), or by amino acid replacements influencing the non-covalent interactions present between the two domains. Evidence for a role of hinge bending in substrate specificity has come from the comparison of the 3-dimensional structures of thermolysin and *B. cereus* NP (Pauptit *et al., 1988;* Stark *et al.,* 1992). The individual domains of both NP's were highly superimposible with only minor differences in the backbone tracing. However, the relative positions of the domains deviated several degrees. This difference can contribute to the somewhat different substrate specificities of the two NP's. In fact, mutants of the *B. subtilis* and *B. stearothermophilus* NP's that were constructed to effect the hinge bending, by substituting Gly residues in the interconnecting α-helix (in particular residue 136 in *B. subtilis* NP), were shown to behave differently on synthetic substrates, when compared to their wild-type counterparts (O.R. Veltman, unpublished results).

Using site directed mutagenesis several structural elements in B. *subtilis* NP have been analysed with respect to their impact on thermostability. An example of a residue that affects stability is the C-terminal amino acid Leu300. Analysis of the 3-dimensional model of the NP (Figure 2.b) suggested that this particular residue is involved in the stabilization of interactions between the two sub domains in the C-terminal domain by occupying a hydrophobic pocket formed by residues from both subdomains. Indeed, deletion of Leu300 or substitution of this amino acid by a smaller (Ala), a polar (Asn), a sterically unfavourable (Ile), or deletion of Leu300, gave rise to NP's with decreased thermostabilities (Table I; Eijsink *et al*., 1990) . It has been shown that thermostability of NP's can be affected by the introduction of disulfide bridges (Table 1; van den Burg *et al*., 1992) In general, disulfide bridges are considered to contribute to stability by decreasing the entropy of the unfolded state. Several examples of the construction of active enzymes with correctly formed disulfide bonds exist. The stability of the mutant enzymes often decreased, due to conformational strain and the effect of the amino acid replacement as such (Wetzel *et al*., 1987; Alber *et al.,* 1989; Pjura *et al*., 1990) leading to irreversible inactivation at elevated temperatures (Wetzel *et al*., 1987; Mitchinson and Wells, 1989; van den Burg *et al*., 1992). In *B. subtilis* NP disulfide bridges have been introduced by replacing amino acid residues Va1102 and Gln120, and Glu186 and Alal94, by cysteines. These positions were selected on the basis of positions of autodigestion target sites determined previously (van den Burg *et al*., 1990). It was anticipated that disulfide bonds close to sites prone to autoproteolysis would make these regions less susceptible by rigidification of local structural elements. However, autodigestion of the enzymes was not notably affected by the newly introduced Cys residues. On the other hand, thermal stability of the variants had decreased dramatically. Structural analyses of the model indicated that the destabilizing effects were caused by replacement of the individual side chains of residues 102, 120, 186 and 194 by cysteine, giving rise to unfavourable local interactions. In particular amino acid Glu186 has an important role in NP with respect to thermal stability as it is involved in Ca²⁺ binding. Calcium ions have been reported to be involved in maintaining the stability of a number of enzymes, e.g. trypsin, α-amylase, glutamine synthetase (Feder et al., 1971). NP's contain 2-4 Ca²⁺ ions, and it has been shown that removal of calcium from thermolysin and *B*. *subtilis* NP is deleterious for stability (Grandi *et al.,* 1980). The role of bound calcium ions was analysed in detail in thermolysin by several authors (Drucker and Borchers, 1971; Roche and Voordouw, 1978; Corbett and Roche, 1983; Fontana *et al.,* 1986). On the basis of biochemical data and analysis of the thermolysin crystal structure, four calcium ions have been identified in thermolysin (Drucker and Borchers, 1971). Circular dichroism of calcium-loaded and calcium-depleted thermolysin indicated conformational changes upon removal of calcium. Fontana *et al.* (1986) showed that these conformational changes correlated well with increased autoproteolysis. The number of bound calcium ions varies between the *Bacillus* NP's. The thermostable variants contain four calcium ions, the thermolabile variants only two, a difference which may underlie the difference in thermostability. Transplantation of a calcium binding loop from thermolysin to *B. subtilis* NP rendered the latter enzyme more thermostable at high Ca²⁺ concentrations (0.1 M). At these concentrations the half-life of denaturation of the mutant NP, as determined by circular dichroism, was significantly higher. At low Ca²⁺ concentrations (1 mM) no effect on stability was observed (Toma *et al*., 1991).

### METHODS

### Plasmids and strains

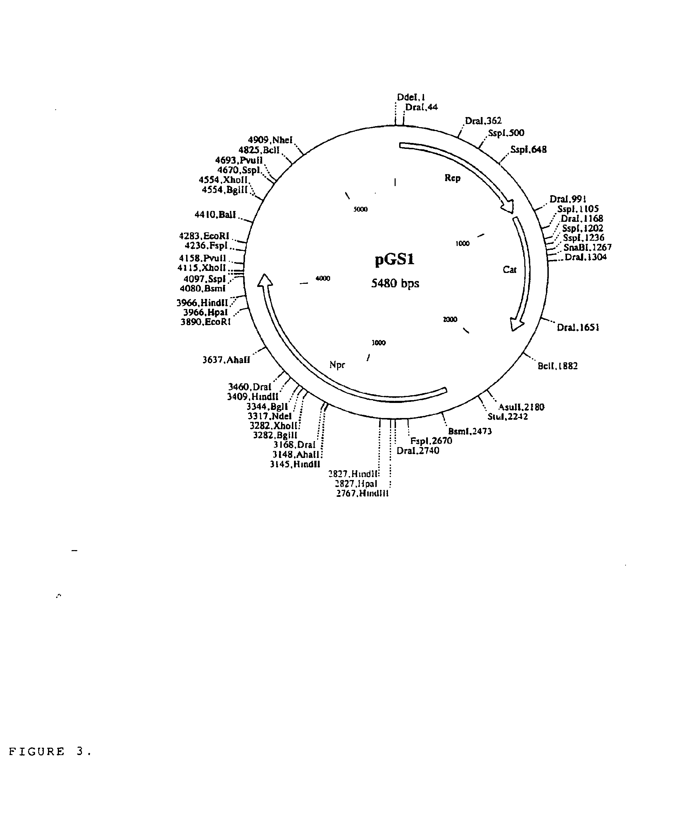

The *B. subtilis npr* gene (originally obtained from L. Mulleners, Gist-Brocades) was subcloned in the high copy number plasmid pTZI2 (Aoki *et al*., 1987) , yielding pGS1 (Figure 3; Eijsink *et al.,* 1990). The gene was expressed in the protease deficient *B. subtilis* strain DB117 (Eijsink *et al*., 1990). Suitable fragments of the *npr* gene were subcloned in the *E. coli* plasmid pMa/c for site-directed mutagenesis (Stanssens *et al*., 1989). *E. coli* WK6 and WK6MutS (Zell and Fritz, 1987) were used in site-directed mutagenesis procedures. All strains were grown on Trypton-Yeast medium, containing the appropriate antibiotics.

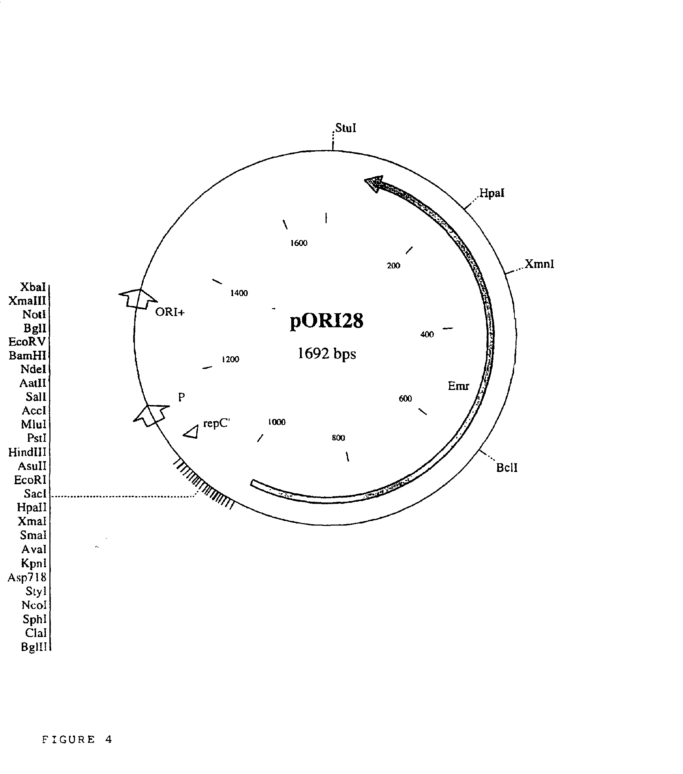

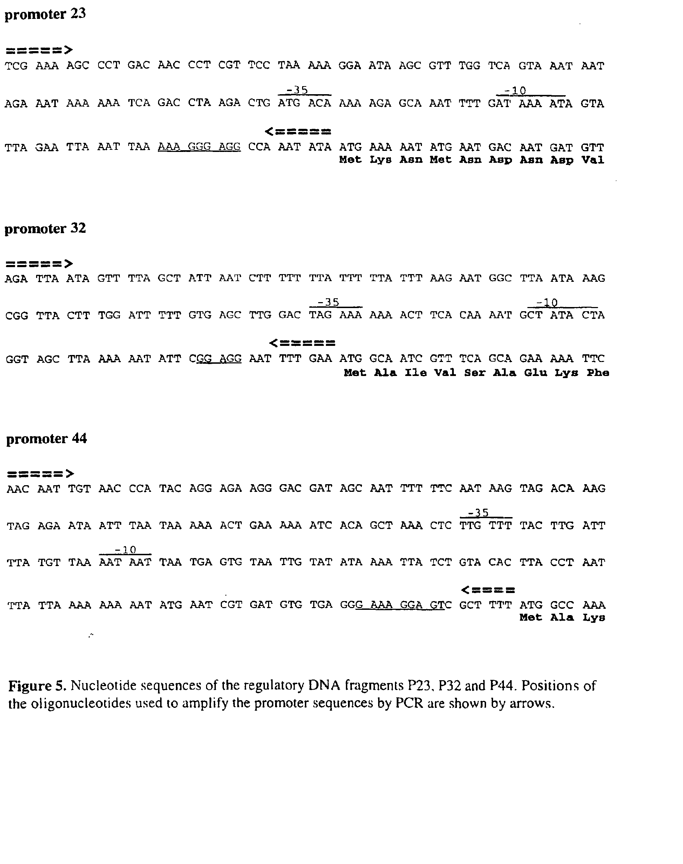

Neutral protease gene variants were cloned in plasmid pORI28 (Figure 4). This plasmid is able to replicate in specific *E. coli, B. subtilis* and *L*. *lactis* strains that express the *repA* gene from their chromosomes. The *npr* gene variants were subcloned using either specific restriction endonucleases or, alternatively, polymerase chain reaction-based methods. The latter approach uses specific oligonucleotides with sequence homology to the start and end of the structural *npr* gene and additional restriction sites for subcloning purposes. After subcloning of the *npr* genes in pORI28, promoter sequences of lactococcal origin (Figure 5) were cloned upstream of the genes, resulting in secretion of active protease by *L. lactis. L. lactis* strains were grown in M17-based media. Protease producing colonies were selected on agar plates supplemented with 0.8% skim milk by their ability to degrade the substrate, which results in a clearing zone around the colonies.

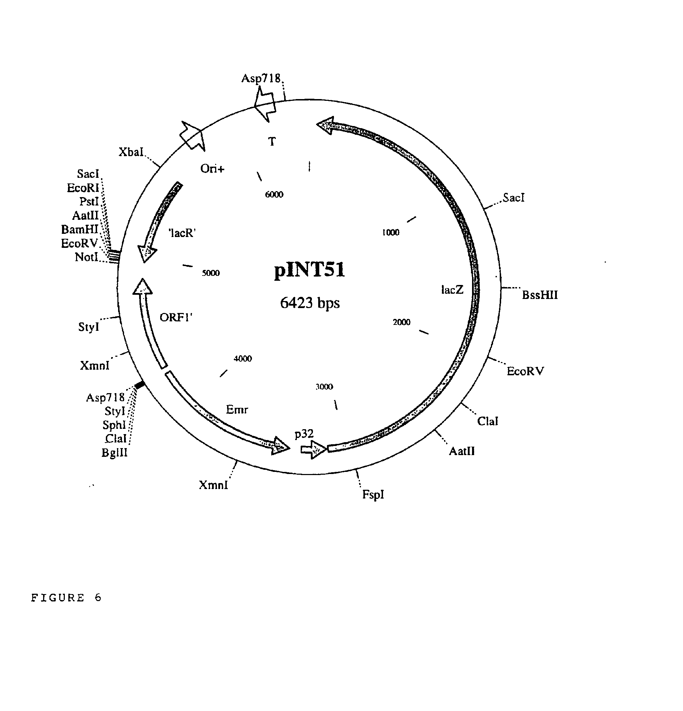

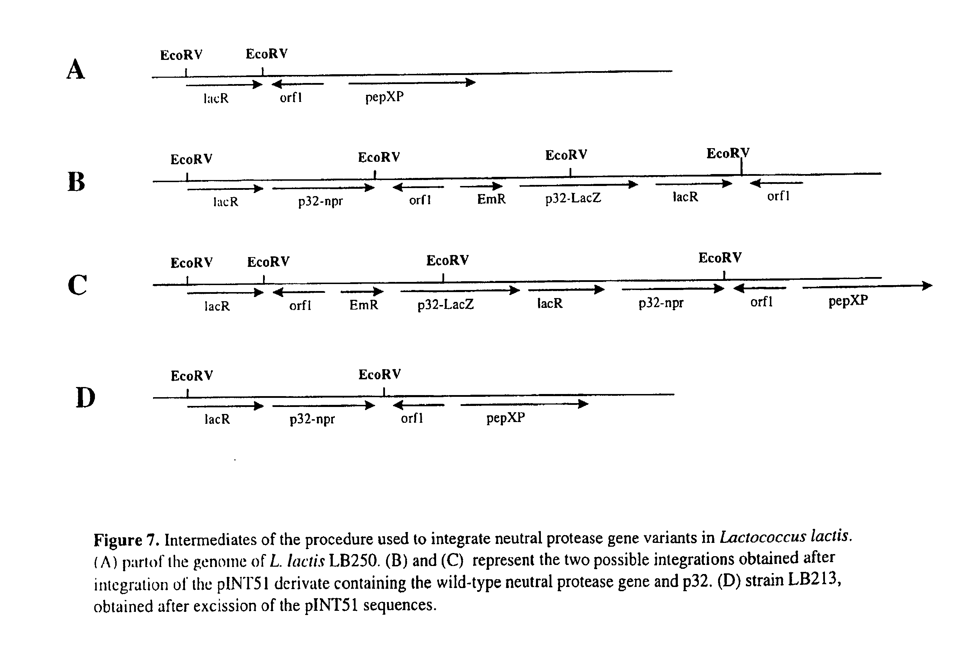

The DNA encompassing *npr* and the lactococcal promoter was isolated from colonies expressing NP and subsequently cloned in pINT51 (Figure 6). pINT51 contains a DNA fragment derived from the chromosome of *L*. *lactis* LB250. In the middle of this fragment several restriction endonuclease recognition sites are available for insertion of the npr/promoter combination. The plasmids thus obtained are not able to replicate in *L. lactis* LB250, but are able to insert into the genome of this strain by homologous recombination. In case a single cross-over event occurs, the whole plasmid becomes part of the lactococcal genome, which can be selected for by the resistance of such strains to the antibiotic erythromycin and to develop blue colonies in the presence of the substrate X-Gal. To eliminate of the antibiotic resistance marker and other plasmid DNA sequences, with the exception of the npr gene, cells are grown for several generations in the absence of erythromycin, resulting in a second recombination event that excises all original pINT51 sequences from the chromosome (Figure 7). Cells in which this has taken place were selected by their inability to become blue in the presence of the chromogenic substrate X-Gal.

### Selection and construction of mutants

The design of destabilizing or activity effecting mutations, theoretical analysis of their structural effects, and structural inspections of the neutral protease molecule was performed using *in computo* procedures described elsewere (Eijsink et *al,* 1990; Vriend and Eijsink, 1993). In these procedures general accepted principles of protein structure and stability were taken into account (e.g. Matthews *et al.,* 1990; Alber *et al.,* 1991; Vriend and Eijsink, 1993, Fersht and Serrano, 1993).

In the mutagenesis procedure mutagenic oligonucleotides used for the production of site-specific mutations were designed such that restriction sites were removed or created, without producing additional changes at the amino acid level. Mutagenesis was performed using a gapped duplex method described earlier (Stanssens *et al.,* 1989). Mutant clones were selected by restriction analysis and their *npr* gene fragments were sequenced using the dideoxy chain termination method (Sanger *et al.,* 1977). For the production of mutant neutral proteases in *B. subtilis,* correctly mutated *npr* gene fragments were used to construct derivates of pGS1 containing an intact *npr* gene.

### Production, purification and characterization of neutral proteases

*B*. *subtilis.* DB117 harbouring a pGS1 variant was cultured in 600 ml medium in aerated 1000 ml flasks at 32°C. After 16 hours of cultivation the cells were removed by centrifugation and the supernatants were loaded onto Bacitracin-silica columns (Unilever Research Laboratories, Vlaardingen, The Netherlands) for affinity chromatography as described previously (Van den Burg *et al.,* 1989) . After purification the enzymes were stored at -18°C in the elution buffer used in the affinity chromatography procedure (20 mM sodium acetate, pH 5.3; 5 mM CaCl₂; 20% (v/v) isopropanol; 2.5 M NaCl; 0.3% sodium azide). Purified enzyme was analyzed using sodium dodecyl sulfate polyacrylamide gel electroforesis (SDS-PAGE) as described before (Van den Burg *et al*., 1989).

Thermostability, expressed as T50, was determined by incubating aliquots of diluted pure enzyme (approx. 0.1 µM in 20 mM sodium acetate, pH 5.3; 5 mM CaCl₂; 0.5% (v/v) isopropanol; 62.5 mM NaCl) at appropriate temperatures. Subsequently, the residual protease activity was determined using a casein assay (Fujii *et al*., 1983). The T50 and δT50 values presented in this invention are derived from at least three independent measurements. The standard deviation in T50 values is approximately 0.7°C; that in δT50 approximately 0.4°C.

Specific activities of wild-type and neutral proteases variants were determined using casein, and the synthetic substrates FAGLA (furylacryl and FAAFA (furylacryl). Kₘ and k_{cat} values were determined using Lineweaver-Burke methodology.

Substrate specificities of wild-type and neutral protease variants were analyzed using β-casein as a substrate. Neutral protease and β-casein were incubated at a enzyme-substrate ratio of 10⁻³ (w:w) in 50 mM sodium acetate, pH 7.5; 5 mM CaCl₂; 50 mM NaCI, and incubated for 16 hours at 30°C. After incubation TFA (trifluoroacetic acid) was added, to a final concentration of 1%, and the TFA-soluble reaction products were separated from the TFA-insoluble products by centrifugation at 13,000 x g. The supernatant was removed and dried by evaporation. Dried peptide preparations were stored at -18°C. Casein degradation was analyzed using HPLC reversed phase chromatography on a Biorad HPLC system. Peptides were separated on a reversed-phase column (250 x 4.6 mm, Hi-Pore 318, Biorad). Solvent A was 0.11% TFA (v/v) and 5% acetonitrile (v/v) in Milli Q water, Solvent B was 0.1% TFA (v/v) and 60% acetonitrile (v/v) in Milli Q water. TFA-soluble peptides were dissolved in solvent A and applied to the column at a flow rate of 1 ml/min. Elution was performed at room temperature using a linear gradient (0-80 %) of solvent B. Peptides were detected by UV absorption at 214 nm.

### Characterization of L. lactis strains expressing neutral protease variants

*L. lactis* strains in which the wild-type *npr* gene with different lactococcal promotor sequences or *npr* gene variants with *L. lactis* promotor P32 had been stable integrated, as described above, were analyzed with respect to growth characteristics, neutral protease production, and proper processing of the gene product.

Growth characteristics of *L. lactis* LB250 and variants thereof expressing the *npr* gene were compared in M17-based medium, supplemented with glucose, and in reconstituted skim milk. Neutral protease production of the variants was compared by determining neutral protease activity in culture supernatants using casein as a substrate. Processing of the neutral protease by LB250 variants was analyzed using anti-neutral protease rabbit antibodies. To 1 ml of overnight culture supernatant 10 µl TFA was added and TFA-insoluble proteins were precipitated by centrifugation. The pellet was dried and resuspended in SDS-PAGE loading buffer. Prior to SDS-PAGE samples were boiled for 5 minutes. After electrophoresis proteins were blotted to nitrocellulose membranes and neutral protease was detected using anti-Npr antibodies according to Towdin and Gordon (1984). Attempts to detect intracellular unprocessed or processed forms of the neutral protease after electrophoresis of cell-free extracts of LB250 variants were hampered by cross-reactivity with proteins from lactococcal origen.

Insertion of npr/promoter sequences in the *L. lactis* genome was analyzed using Southern hybridization techniques, essentially according to Maniatis (1982). Chromosomal DNA was isolated from *L. lactis* and subsequently digested with selected restriction endonucleases. After separation of the resulting fragments by agarose gel electrophoresis, DNA was blotted to nitrocellulose membranes. Recombination was visualized using *npr* gene fragments as probes. Probes were labelled using the ECL procedure (Amersham, Buckinghamshire, UK). Detection was performed by a fluorescence technique (ECL, Amersham, Buckinghamshire, UK), according to the supplier. Parts of pINT51 were used as probes to ascertain the removal of vector sequences from the *L*. *lactis* variants genomes after the second recombination event.

### EXAMPLE 1

### Construction of L. lactis variants expressing different levels of the B. subtilis neutral protease

The *B. subtilis* neutral protease gene was amplified by polymerase chain reaction (PCR) using the synthetic oligonucleotides listed in Table 2 and plasmid pGS1 (Figure 3) as a template.

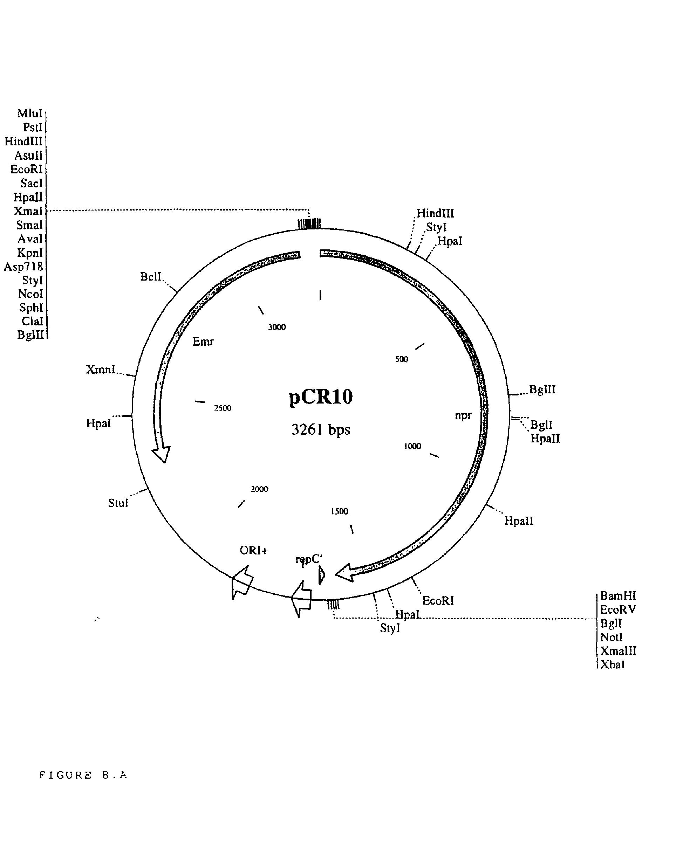

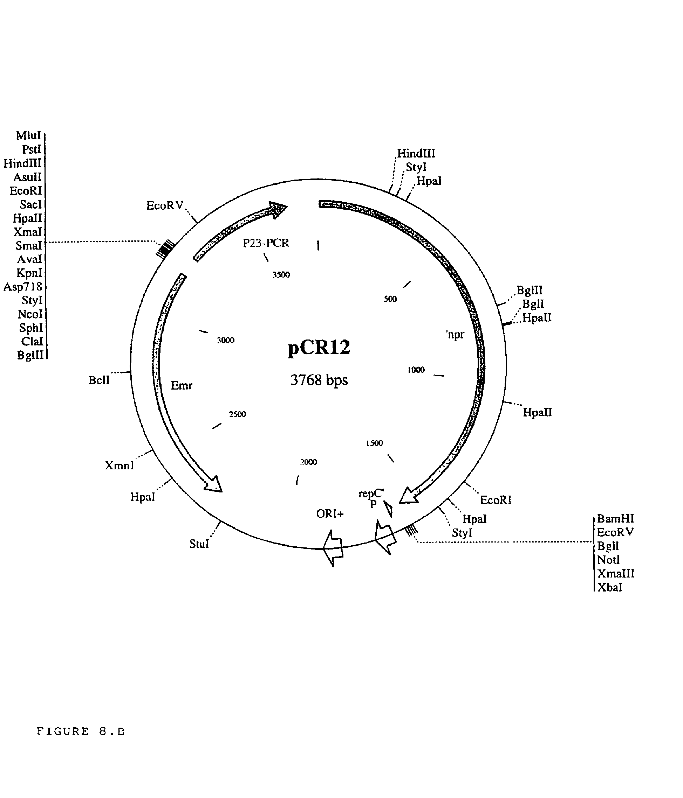

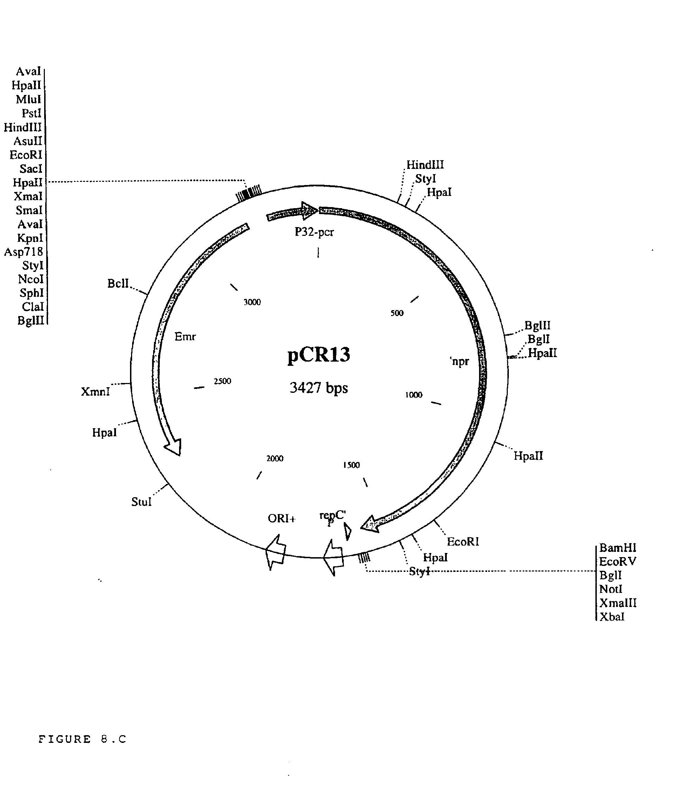

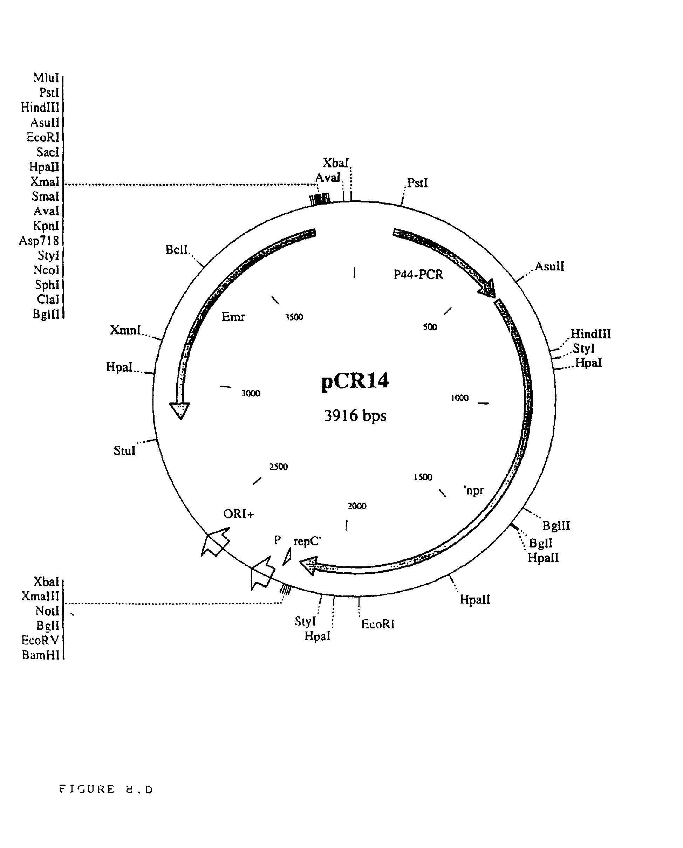

The resulting 1700 bp DNA fragment contains the entire *npr gene,* including the GTG start codon and the TAA stopcodon. Upstream the startcondon an additional 16 bp fragment is present, that contains the recognition sequences for the restriction endonucleases MluI, SalI and PmlI. Downstream the stop codon 16 bp are present that contain recognition sequences for BamHI and SalI. After purification of the PCR product the DNA fragment was digested with BamHI and MluI and, subsequently, ligated in pORI28 (Figure 4) that had been digested with the same restriction enzymes. Next, the ligation mixture was transformed to *E. coli* EC1000, a strain that contains a copy of the *repA* gene on the chromosome, thus enabling replication of pORI28 derivates, and selected on TY plates supplemented with 100 µg/ml erythromycin. DNA from transformants was analyzed by restriction analysis and transformants harbouring pCR10 (Figure 8; pORI28 with the *B. subtilis npr* gene) were selected for further constructions. Due to the lack of promoter sequences upstream the *npr* gene no protease production was present in these transformants. To obtain NP production lactococcal promoters were cloned upstream the gene in pCR10. For this purpose three promoter sequences were selected; P23, P32 and P44, respectively (Figure 5, van der Vossen *et al.,* 1987). Promoter sequences were obtained by PCR, using the oligonucleotides listed in Table 2, and pGKV223, pGKV232 and pGKV244 (van der Vossen *et al*., 1987). Using the chloramphenicol gene as a reporter relative activities were determined to be 3.2, 0.6 and 0.2 for P23, P32 and P44, respectively. The 3'-oligonucleotides were chosen such that the original ORF sequences downstream of the promoters were removed after PCR. After amplification and purification of the promoter fragments they were inserted in pCR10, upstream the *npr* gene, yielding pCR12, pCR13 and pCR14, resp (Figure 8). For that reason, pCR10 was digested with PmlI and MluI. The P23 and P32 fragments were digested with NsiI, made blunt with T4 DNA polymerase and subsequently digested with MluI. The P44 fragment was digested with HindIII, treated with T4 DNA polymerase and thereafter digested with MluI. The different fragments were purified, ligated and electrotransformed to *L. lactis* 108 (repA⁺). The transformation mixture was plated on M17 agar, supplemented with 0.5% glucose, 1% succrose, 5 µg/ml erythromycin, and 0.8% skim milk. The latter addition facilitates the detection of protease producing colonies, by the ability of the protease to digest milk proteins, resulting in a clear zone around the colony. Protease-positive transformants were selected and the plasmid DNA was analyzed by restriction analysis. From pCR12, pCR13 and pCR14 promoter/npr fragments were isolated after digestion with NotI and PstI. These fragments were ligated with pINT51 digested with the same restriction enzymes, yielding pCR112, pCR113 and pCR114 (Figure 8). After electrotransformation these constructs were selected for on M17 agar as described above, additionally supplemented with chromogenic substrate x-gal (0.006%). DNA from protease proficient blue colonies was further analyzed by restriction analysis. DNA from pCR112, pCR113 and pCR114 was electrotransformed to *L. lactis* LB250, followed by selection on M17 plates containing erythromycin, skim milk and X-gal. Due to the fact that *L. lactis* LB250 does not contain the repA gene, erythromycin-resistant, halo-forming and blue-colouring colonies must be the result of a recombination event, in particular a single cross-over (Figure 7).

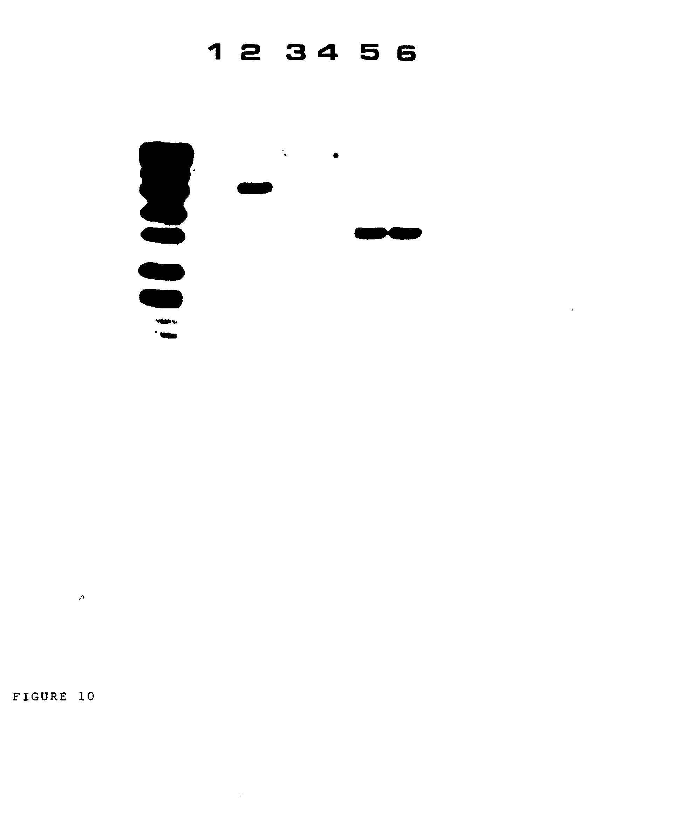

This recombination event was analyzed by Southern hybridization. Total chromosomal DNA from positive clones was digested and after electrophoresis transferred to nitrocellulose. Presence and orientation of pCR112, pCR113 and pCR114 DNA was determined using the *npr* gene or parts of pINT51 as probes. Clones in which a proper recombination had taken place were subsequently grown for approximately 100 generations without selecting for erythromycin resistance. This results in loss of part or all of the integrated sequences by a second recombination event. Ideally, the pINT51 sequences are lost, whereas the promoter/npr sequences retain in the chromosome. This situation can be selected for by screening for white colonies (due to the loss of the *lacZ* gene) that are still able to form halo's. Several of the clones thus obtained were characterized in detail by Southern hybridization. Hybridization with *npr* as a probe yielded a clear signal (Figure 10), whereas a probe from pINT51 was not able to hybridize. Strains, positive with respect to these analyses, were designated LB212, LB213 and LB214 for containing the *npr* gene preceded by P23, P32 and P44 respectively. Since these strain do not contain any vector sequences, nor antibiotic resistance genes, they can be considered as "food-grade".

### Characterization of L. lactis LB212, LB213 and LB214

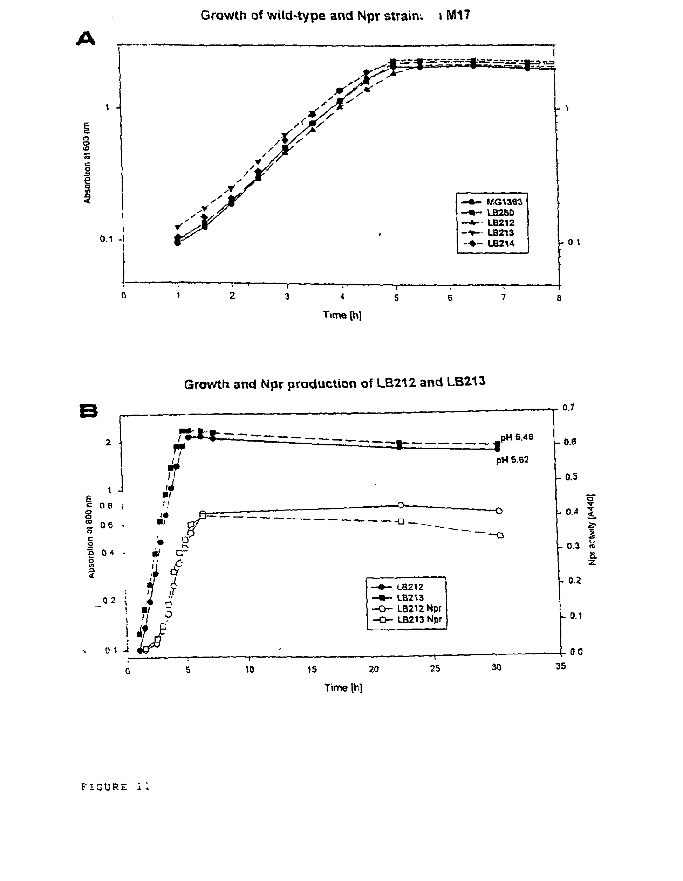

Strains in which the *B. subtilis npr* gene had been integrated were analyzed with respect to growth and protease processing and production. Growth characteristics in M17-based medium were shown to be similar for the wild-type LB250 and the three modified strains tested (Figure 11). Growth in milk

Protease production was determined on M17 plates supplemented with skim milk. Whereas LB250 is not able to form halo's on such plates, LB212, LB213 and LB214 colonies were able to degrade the milk in the plates, resulting in halo formation. The size of these halo's can be correlated to the production levels of the protease. Comparison of the halo sizes suggested similar production levels in LB212 and LB213, whereas production by LB214 is significantly lower.

Protease production was analyzed in culture supernatants also. For that purpose, 150 µl from the supernatants of early stationary phase cultures were incubated with 250 µl of azocasein in 50 mM Tris-HC1, pH 7.5; 5 mM CaCl₂, 0.08% NaN₃. After incubation for 19 hours at 37°C the reaction was stopped by addition of 1.25 ml 10% TCA. After precipitation of the acid-insoluble fraction, 0.9 ml of the supernatant was added to 1.05 ml 1M NaOH and the absorbance at 440 nm was measured. The amount of peptides released is a measure of the proteolytic activity of the samples. The protease activities of the different strains are listed in Table 3, and show that the amounts of protease secreted by LB212 and LB213 are similar, and several times higher than that of LB214. The amounts of the strains LB282, LB292 and LB297 are about half of that of LB212 and LB213.

Proteins from overnight cultures (2 ml) were precipitated by addition of TFA to a final concentration of 1% (v/v). Precipitated proteins were collected by centrifugation, dried and dissolved in 50 µl SDS-PAGE loading buffer. 20 µl was used for SDS-PAGE and after electrophoresis the proteins were electrotransferred to nitrocellulose membranes. Using anti-NP rabbit serum bands migrating at the position of the mature form of the NP, as produced by *B. subtilis,* were detectable in the samples derived from LB212 and LB213. In the sample from LB214 no signal was detectable, which might be caused by the low production levels of the latter strain.

### EXAMPLE 2

### Construction of L. lactis strains expressing B. subtilis neutral protease variants with decreased stabilities.

*B. subtilis* neutral protease variants that show decreased thermal stabilities were selected to be integrated in the genome of *L*. *lactis.* These variants were previously constructed and tested in *B. subtilis.* Analyses of the variants showed that thermostability had decreased dramatically as the result of mutation the C-terminal residue Leu300 or by introducing disulfides between residues 102-120 and 186-194 (Table 1).

The genes encoding the NP variants were amplified by PCR using the oligonucleotides listed in Table 2. For the residue 300 variants the wild-type *npr* gene was the template and oligonucleotides introducing the substitutions or deletion were used in combination with oligonucleotide NP-5'. For the amplification of the disulfide variants, the genes already containing these changes, as present in *B. subtilis,* were used as templates for oligonucleotides NP-5' and NP-3'. The residue 300 variant PCR products were made blunt with T4 DNA polymerase and subsequently digested with MluI. The resulting fragment was ligated with pORI28 digested with EcoRV and MluI. The disulfide variants PCR products were digested with BamHI and MluI and ligated with pORI28 that had been digested with the same enzymes. After selection of the proper constructs, P32 was inserted in these constructs as described above. Using this strategy the following constructs were obtained: pCR22 (Leu300Ala); pCR32 (Leu300Ile); pCR42 (Leu300Asn); pCR52 (Leu300del); pCR62 (Va1102Cys-G1n120Cys); pCR72 (Glu186Cys-Ala194Cys).

The constructs mentioned above gave rise to production of extracellular protease in *L*. *lactis* 108, as visualized by halo formation on plates supplemented with skim milk, except for the constructs pCR52 and pCR72. For the latter two mutants it is known that expression of these mutants in *B. subtilis is* decreased also (B. van den Burg, V.G.H. Eijsink, unpublished results).

After transfer of the *npr* gene variants to pINT51, the genes can be transferred to *L. lactis* LB250. After correct and stable integration protease activities of the different constructs can be compared. Strains with desirable expression levels can subsequently be used in cheese manufacturing experiments.

### EXAMPLE 3

### Construction of L. lactis strain expressing B. subtilis neutral protease variants with altered specificity

The use of *B. subtilis* NP variants as expressed by *L. lactis* might be beneficial, since such enzymes degrade the milk proteins differently. This might add to the development of diary products with improved or new characteristics. Neutral protease variants were selected that, as the result of single amino acid substitutions, behave differently towards protein substrates. Firstly, a NP variant was constructed that had residue Glnl20 substituted by Cys, using site-directed mutagenesis. This residue is located near the entrance of the active site and it was anticipated that, by changing the character of this residue, penetrating of substrates would be affected. The mutant was constructed using a synthetic oligonucleotide (Table 2), essentially according to Stanssens *et al.* (1989). The mutated gene was expressed in *B. subtilis* and the gene product was purified (van den Burg *et al.,* 1989) and
characterized. A second NP variant was constructed in which Phe134 was substituted by Leu. Inspection of the 3-D model of NP suggested that this particular residue might be involved in substrate binding. Changing the size of this residue could have effect on the substrate specificity of the NP. The substitution was accomplished with a synthetic oligonucleotide (Table 2) and the gene was reintroduced and expressed in *B*. *subtilis.* Thirdly, Gly136 was substituted by Ala. Since residue 136 is located in the α-helix connecting the N- and C-terminal domains, it was anticipated that by introduction of a larger side chain the hinge-bending, being the observed motion between the domains, could be decreased. The substitution was constructed as described above (see Table 2 for oligonucleotide used).

The NP variants were produced by *B. subtilis* and purified from the culture supernatants by affinity chromatography (van den Burg *et al.,* 1989). Purification yields and purity were analyzed by SDS-PAGE. Yields and purities were similar to the wild-type except for the variant Glnl20Cys. In the latter case high molecular weight aggregates, caused by intermolecular disulfide bond formation, were visible during SDS-PAGE in the absence of reducing agent. However, it was shown that this aggregation was caused by boiling the samples prior to electrophoresis, since it was absent in case the samples were heated at lower temperatures. -

Specificic activities of the variants were determined using casein and the synthetic substrates FAGLA (N-(3-[2-furyl]acryloloyl)-Gly-Leu amide) and FAAFA (N-(3-[2-furyl]acryloloyl)-AlA-Phe amide) as substrates.

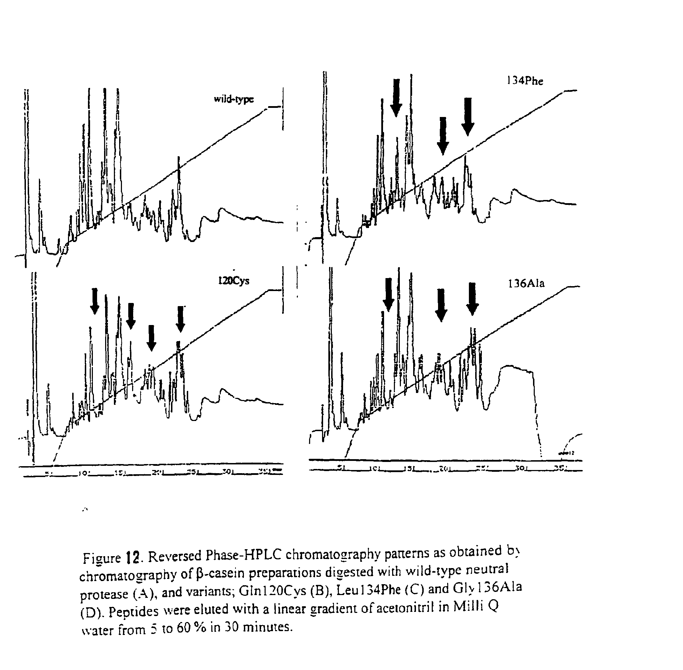

Substrate specificity was analysed and compared to the wildtype NP using β-casein as model substrate. Enzyme and substrate were incubated at a ratio of 10⁻³ for 16 hours at 30°C. After separation of the acid soluble and insoluble peptides, the reaction products were analyzed using Reversed Phase-HPLC. As becomes evident from Figure 12, substrate specificity towards β-casein is effected by substituting residues 120, 134 and 136. After introduction of the gene encoding these variants into the chromosome of *L. lactis,* using the procedure described in Example 1, contribution of these variants to the flavour development in dairy products can be tested. Furthermore, new dairy products might be the result from the use of variants such as those discussed above. Neutrol protease production levels of the resulting strains LB282, LB292 and LB297 were determined and fond to be similar to those of LB212 and LB213 (Table 3).

### EXAMPLE 5

### production of laboratory scale cheeses using bacteria provided with neutral protease activity according to the invention ((Testing L. lactis LB212, LB213 and LB214 in cheese production).

Two Gouda type rindless cheeses of 175g were prepared for each different set of parameters of the experiment as given in table 4. To the milk of the reference experiment Calcium Chloride and rennet was added together with 0.6% of starter culture Bos, an often used production start of culture. After coagulation and cutting 50% of the whey was removed and 30% rinsing water was added. After 50 min.stirring the curd was separated from the whey and subsequently pressed in cheesemoulds. A further reference experiment included the addition of 0.1 to 0.4% calculated on the milk of the mother microorganism culture besides the starter culture. The experiments employing neutral protease activity according to the invention comprised the same addition of a microorganism of the mother culture, but than genetically modified as disclosed in table 5. The cheese production process was the same throughout the set of experiments. The resulting cheeses were put in brine for several hours and subsequently wrapped in Cryovac foil. They were allowed to ripen at a temperature of 7 degrees Celsius. After three, respectively 12 weeks of ripening the cheeses were analysed for composition, i.e. moisture content, fat content, salt content and pH. Nitrogen analyses of the cheeses (total N), aqueous extracts (soluble N) of the cheeses and cheeses after aqueous extraction (amino N)were also performed. The results are given in table 4.

### FIGURE LEGENDS

***Figure 1.a**.* Alignment of the primary sequences of neutral proteases from *Bacillius thermoproteolyticus (1), B. stearthermophilus (2), B . cereus (3), B . Subtilis* (4) and *B. amyloliquefaciens (5).*
***Figure 1.b**.* Nucleotide sequence of the mature part of the neutral protease gene from *B. subtilis* and the drerived amino acid sequence. Positions and character of amino acid substitutions are shown in bold.
***Figure* 2**. Three-dimensional models of thermolysin (A) and *B. subtilis* neutral protease (B). Active site of thermolysin is indicated by an arrow. The dotted circle represents the zinc ion, the filled-in circles indicate calcium ions.
***Figure 3**.* Plasmid pGS1, containing the wild-type *B. subtilis* neutral protease.
***Figure 4**.* Plasmid pORI28
***Figure* 5**. Nucleotide sequences of regulatory DNA fragments P23, P32 and P44. Positions of the oligonucleotides used to amplify the promotor sequences by PCR are shown.
***Figure* 6**. Plasmid pINT51
***Figure 7***. Intermediates of the procedure used to integrate neutral protease gene variants in *Lactococcus lactis.(A)* part of the genome of *L. lactis* LB250.(B) and (C) represent the two possible integrations obtained after integration of the pINT51 derivate containing the wild-type neutral protease gene and P32.(D) strain LB213, obtained after excission of the pINT51 sequences.
***Figure 8**.* Plasmids pCR10, pCR12, pCR13 and pCR14.

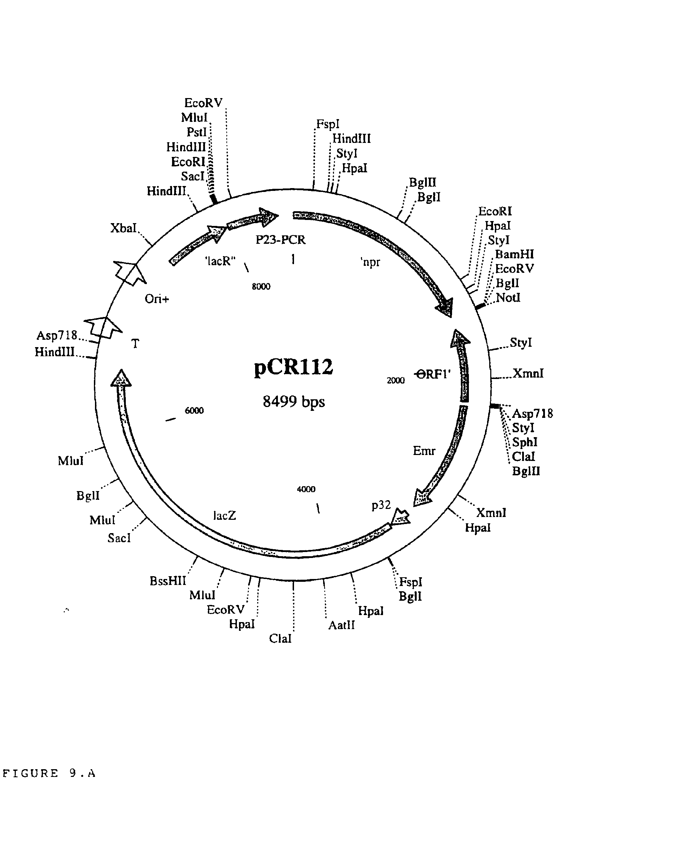

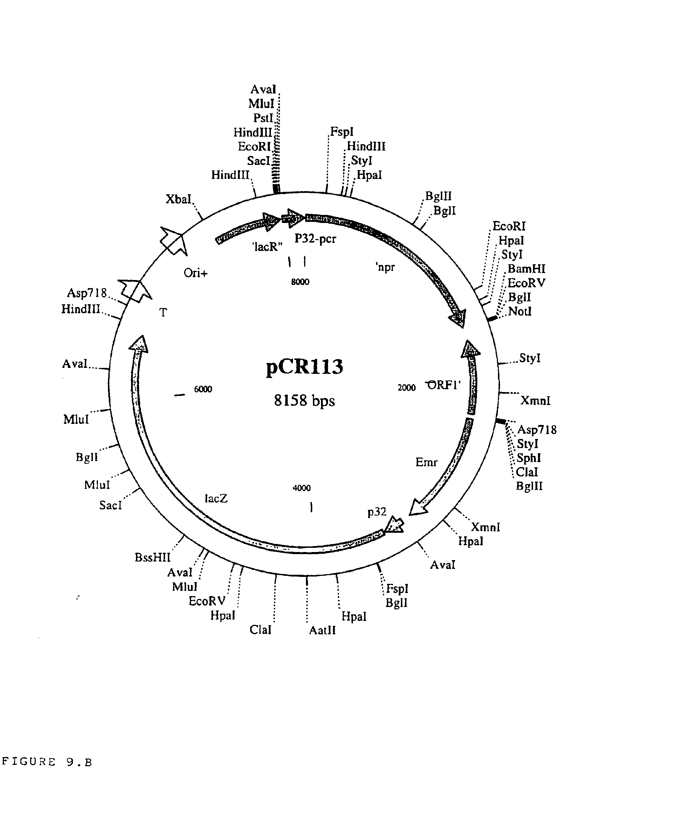

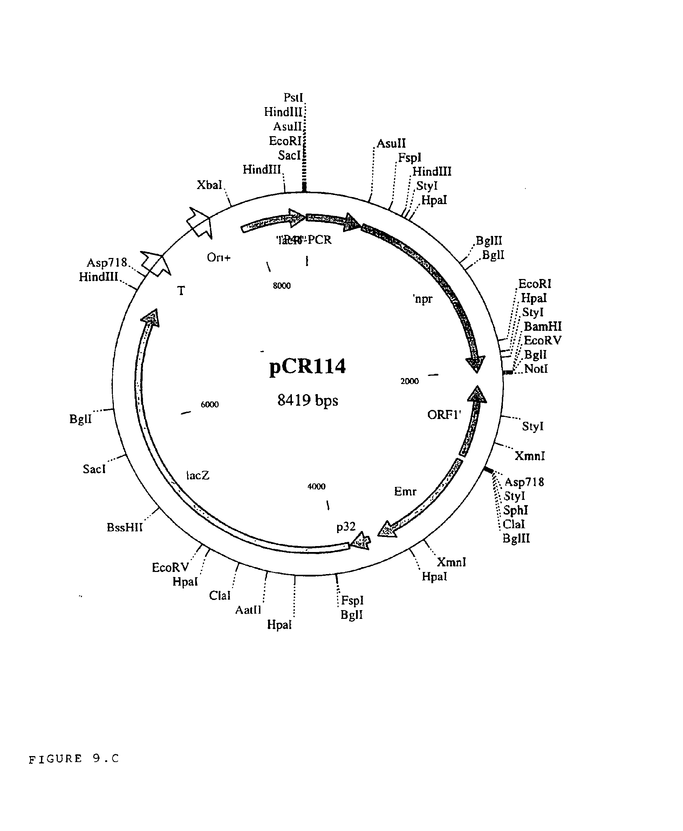

***Figure 9**.* Plasmids pCR112, pCR113 and pCR114.
***Figure 10***. Southern hybridization analysis of the construction of LB213. The *B. subtilis* neutral protease gene was used as a probe. In all lanes EcoRV digested chromosomal DNA was loaded from respectively, (1) LB250; (2) strain LB113, wherein pCR113 has been integrated (see Figure 7.C); (3) and (4) strain from which the complete pCR113 sequence was removed by homologous recombination, yielding LB250 again; (5) and (6) strain LB213, obtained arter excission of the pINT51 sequences (see also Figure 7.D).
***Figure 11.*** Growth curvesof wild-type *L.lactis* strains and variants expressing the *B.subtilis npr* gene (A), and growth and neutral protease production of strains LB212 and LB213 (B).
***Figure 12.*** Reversed phase-HPLC chromatography patterns as obtained by chromatography of β-casein preparations digested with wild-type neutral protease (A) and variants; Gln120Cys (B), Phe133Leu (C), Gly135Cys (D). Peptides were eluted with a lineair gradient of acetonitril in Milli Q water from 5 to 60% in 30 minutes.

**Table 1**

| *Thermal stabilities of B. subtilis neutral protease variants* | | |
|---|---|---|
| neutral protease | T50 (°C) | δT50 |
| wild-type | 58 | 0 |
| Leu300Ala | 48 | -10 |
| Leu300Ile | 50 | - 8 |
| Leu300Asn | 45 | -13 |
| Leu30Odel | 42 | -16 |
| VallO2Cys/Glnl20Cys | 52 | - 6 |
| Glul86Cys/Alal94Cys | 48 | -10 |

T50 is the temperature at which 50% of the activity is lost in 30 minutes.

**Table 2. synthetic oligonucleotides used for PCR**

| name | sequence | characteristics |
|---|---|---|
| NP-5' | AAAACGCGTCGACCACGTGGGTTTAGGTAAG | start npr gene, MluI, SalI, PmlI |
| NP-3' | CGCGGATCCGTCGACATTACAATCCAACAGCATTCCAGGC | stop npr gene, BamHI, SalI |
| P23-5' | TTCGAACTGCAGACGCGTGATCGCATCATTTTC | "start" P23, BstBI, PstI, MluI |
| P23-3' | AAAGCGATGCATATATTTGGCCTCCC | "end" P23, NslI |
| P32-5' | TTCGAACTGCAGACGCGTCCGGTCCTCGGG | "start" P32, BstBI, PstI, MluI |
| P32-3' | AAAGCGATGCATTCAAAATTCCTCC | "end" P32, NsiI |
| P44-5' | TTGGAAACGCGTCAGAACGATGAAAAAAG | "start" P44, BstBI, MluI |
| P44-3' | AAAGCTTCGAAAAGCGACTCCTTTC | "end" P44, HindII, BstBI |
| Leu300Ala | CTAATATCACGCTCCAACAGC | replaces Leu300 by Ala, SspI- |
| Leu300Ile | CTAATATTAAATTCCAACAGCGTTCCAGGC | replaces Leu300 by Ile, BsmI- |
| Leu300Asn | CTAATATTAATTTCCAACAGCATTCC | replaces Leu300 by Asn, AsnI+ |
| Leu300del | CTAATATTACAATCCAACAGCGTTCCAGGC | deletes Leu300, BsmI- |
| Val102Cys | GTGCACGGAAGAACAGATTTTACTGCC | replaces Val102 by Cys, SnoI+ |
| Gln120Cys | CCGTAAATCATGCAGTCTCCGGTCC | replaces Gln120 by Cys, AvaII+ |
| Glu186Cys | CCGTAATGTGGCAACCGATATCCC | replaces Glu186 by Cys, EcoRV+ |
| Ala194Cys | GGACAAGCTTCGAAGACAAGGCTGGC | replaces Ala194 by Cys, HindIII+ |
| Phe134Leu | TAATGAGCCGGAGAACGGAGAGAA | replaces Phe134 by Leu |
| Gly136Ala | CACATCTAATGACGCGGAAAGCGG | replaces Gly136 by Ala |

**Table 3. Extracullar neutral protease activities of different promotor contructs and different specificity variants. Activity was determined using the azo-casein (A440) assay.**

| STRAIN | Absorbance at 440 nm |
|---|---|
| | |
| LB250 (Npr-) | 0 |
| LB212 (P23-Npr) | 0.325 |
| LB213 (P32-Npr) | 0.352 |
| LB214 (P44-Npr) | 0.022 |
| LB282 (P32-Npr120Cys) | 0.221 |
| LB292 (P32-Npr134Phe) | 0.191 |
| LB297 (P32-Npr136Ala) | 0.168 |

**Table 4**

| cheese starter | | pH | moisture (%) pasteurized cheese | salt | TH | | SH/TH | | AN/TH | |
|---|---|---|---|---|---|---|---|---|---|---|
| | 3 weeks | 12 weeks | mean | mean | 3 weeks | 12 weeks | 3 weeks | 12 weeks | 3 weeks | 12 weeks |
| reference 1 | 5.41 | 5.56 | 40.8 | 3.1 | 3.8 | 3.9 | 4.4 | 10.7 | 3.2 | 9.1 |
| reference 2 | 5.35 | 5.46 | 42.1 | 3.5 | 3.8 | 3.8 | 4.5 | 10.7 | 3.5 | 9.1 |
| reference 3 | 5.36 | 5.45 | 41.0 | 3.1 | 3.9 | 3.8 | 4.0 | 11.4 | 3.0 | 8.5 |
| reference 4 | 5.37 | 5.40 | 42.1 | 3.7 | 3.6 | 3.8 | 4.4 | 12.0 | 3.5 | 9.6 |
| reference 5 | 5.29 | 5.40 | 42.5 | 3.5 | 3.7 | 3.8 | 4.5 | 11.8 | 3.2 | 8.8 |
| LB250 0.4% | 5.31 | 5.42 | 43.6 | 3.8 | 3.7 | 3.7 | 4.6 | 10.8 | 3.8 | 8.8 |
| LB250 0.1% | 5.22 | 5.39 | 42.6 | 3.4 | 3.7 | 3.7 | 4.4 | 9.4 | 3.2 | 6.8 |
| LB250 0.4% | 5.38 | 5.53 | 42.2 | 3.4 | 3.8 | 3.8 | 4.5 | 9.0 | 3.4 | 7.5 |
| LB212 0.4% | 5.33 | 5.44 | 42.0 | 3.1 | 4.1 | 3.8 | 8.5 | 24.7 | 5.7 | 20.1 |
| LB212 0.1% | 5.30 | 5.41 | 41.8 | 3.2 | 3.8 | 3.7 | 4.3 | 14.6 | 3.4 | 11.9 |
| LB212 0.4% | 5.42 | 5.65 | 41.0 | 3.0 | 3-8 | 4.0 | 5.8 | 17.7 | 4.3 | 14.3 |
| LB212 0.1% | 5.37 | 5.57 | 40.8 | 3.0 | 3.8 | 3.9 | 4.4 | 14.4 | 3.2 | 11.8 |
| LB213 0.4% | 5.29 | 5.55 | 43.5 | 3.6 | 3.6 | 3.8 | 7.7 | 17.9 | 5.4 | 14.0 |
| LB213 0.1% | 5.28 | 5.50 | 43,7 | 3.7 | | 3-73.6 | 5.4 | 12.1 | 4.3 | 10.0 |
| L8214 0.4% | 5.33 | 5.47 | 42.2 | 3.2 | 3.8 | 3.8 | 3.9 | 11.8 | 3.1 | 9.8 |
| LB214 0.1% | 5.23 | 5.26 | 41.9 | 3.3 | 3.7 | 3.6 | 4.5 | 10.8 | 3.6 | 8.7 |
| LB215 0.4% | 5.36 | 5.52 | 42.6 | 3.6 | 3.7 | 3.7 | 4.5 | 11.5 | 4.0 | 8.8 |
| LB215 0.1% | 5.39 | 5.44 | 42.7 | 3.7 | 3.6 | 3.6 | 4.6 | 12.0 | 3.5 | 9.3 |

## Claims

1. A method for carrying out a process of biotransformation of a substrate whereby at least one lactic acid bacterium is used, said bacterium comprising a gene encoding a Bacillus subtilis neutral protease variant having an altered resulting activity.

2. A method according to claim 1 whereby at least one neutral protease has an altered stability or specificity.

3. A method according to claim 1 or 2, whereby the altered resulting activity is accomplished by regulation of expression of at least one neutral protease gene.

4. A method according to anyone of claims 1-3, whereby the biotransformation process is fermentation of a food product.

5. A method according to claim 4 whereby the substrate is milk or a milk derivative.

6. A method according to claim 5 whereby the biotransformation process is a part of a cheese making process.

7. A method according to any one of claims 1-6 whereby the gene comprises at least one of the following mutations: mutation of the codon encoding leucine at position 300;
: deletion of the codon encoding leucine at position 300;
: mutation of the codons for residues 120, 134 and/or 136;
: mutation of the codons 186 and 194 to cysteine residues; or
: mutation of the codons 102 and 120 to cysteine residues.

8. A method according to anyone of the aforegoing claims, whereby the lactic acid bacterium is a Lactococcus species.

9. A method according to anyone of the aforegoing claims whereby the gene encoding the neutral protease is integrated into the genome of the lactic acid bacterium without residual transfecting material.

10. A food product obtainable by a method according to any one of the aforegoing claims.

11. A food product according to claim 10 which is cheese.

## Patentansprüche

1. Verfahren zur Durchführung eines Verfahrens zur Biotransformation eines Substrats, wobei mindestens ein Milchsäurebakterium verwendet wird, wobei das Bakterium ein Gen umfaßt, codierend eine Bacillus subtilis neutrale Proteasevariante mit einer veränderten resultierenden Aktivität.

2. Verfahren gemäß Anspruch 1, wobei mindestens eine neutrale Protease eine veränderte Stabilität oder Spezifität aufweist.

3. Verfahren gemäß Anspruch 1 oder 2, wobei die veränderte resultierende Aktivität durch Regulation der Expression von mindestens einem neutralen Proteasegen bewirkt wird.

4. Verfahren gemäß einem der Ansprüche 1 bis 3, wobei das Biotransformationsverfahren die Fermentation eines Nahrungsmittelprodukts ist.

5. Verfahren gemäß Anspruch 4, wobei das Substrat Milch oder ein Milchderivat ist.

6. Verfahren gemäß Anspruch 5, wobei das Biotransformationsverfahren ein Teil eines Käseherstellungsverfahrens ist.

7. Verfahren gemäß einem der Ansprüche 1 bis 6, wobei das Gen mindestens eine der folgenden Mutationen umfaßt: Mutation des Codons, codierend Leucin an Position 300;
: Deletion des Codons, codierend Leucin an Position 300;
: Mutation der Codons für die Reste 120, 134 und/oder 136;
: Mutation der Codons 186 und 194 zu Cysteinresten oder
: Mutation der Codons 102 und 120 zu Cysteinresten.

8. Verfahren gemäß einem der vorstehenden Ansprüche, wobei das Milchsäurebakterium eine Lactococcus-Art ist.

9. Verfahren gemäß einem der vorstehenden Ansprüche, wobei das Gen, codierend die neutrale Protease in das Genom des Milchsäurebakteriums ohne restliches Transfektionsmaterial integriert ist.

10. Nahrungsmittelprodukt, erhältlich durch ein Verfahren gemäß einem der vorstehenden Ansprüche.

11. Nahrungsmittelprodukt gemäß Anspruch 10, wobei es sich um Käse handelt.

## Revendications

1. Procédé pour mettre en oeuvre un processus de biotransformation d'un substrat, dans lequel au moins une bactérie lactique est utilisée, ladite bactérie comprenant un gène codant un variant de protéase neutre de Bacillus subtilis ayant une activité résultante altérée.

2. Procédé selon la revendication 1, dans lequel au moins une protéase neutre a une stabilité ou une spécificité altérée.

3. Procédé selon la revendication 1 ou 2, dans lequel l'activité résultante altérée est obtenue par la régulation de l'expression d'au moins un gène de protéase neutre.

4. Procédé selon l'une quelconque des revendications 1-3, dans lequel le processus de biotransformation est la fermentation d'un produit alimentaire.

5. Procédé selon la revendication 4, dans lequel le substrat est du lait ou un dérivé du lait.

6. Procédé selon la revendication 5, dans lequel le processus de biotransformation fait partie d'un processus de fabrication de fromage.

7. Procédé selon l'une quelconque des revendications 1-6, dans lequel le gène comprend au moins une des mutations suivantes :
mutation du codon codant la leucine en position 300 ;
délétion du codon codant la leucine en position 300 ;
mutation des codons pour les résidus 120, 134 et/ou 136 ;
mutation des codons 186 et 194 en résidus cystéine ; ou
mutation des codons 102 et 120 en résidus cystéine.

8. Procédé selon l'une quelconque des revendications précédentes, dans lequel la bactérie lactique est une espèce de Lactococcus.

9. Procédé selon l'une quelconque des revendications précédentes, dans lequel le gène codant la protéase neutre est intégré dans le génome de la bactérie lactique sans matériel de transfection résiduel.

10. Produit alimentaire pouvant être obtenu par un procédé selon l'une quelconque des revendications précédentes.

11. Produit alimentaire selon la revendication 10, qui est un fromage.
